# EUROPEAN PATENT SPECIFICATION

(11) **EP 3 970 782 B1**
(45) Date of publication and mention of the grant of the patent: **10.09.2025**
(21) Application number: 19931574.8
(22) Date of filing: 14.11.2019
(51) Int. Cl.: A61M 25/10, A61F 7/12

(54) **BALLOON CATHETER**
BALLONKATHETER
CATHÉTER À BALLONNET

(30) Priority: 06.06.2019 CN 201910492068
(43) Date of publication of application: 23.03.2022
(73) Proprietor: Piedmont Medsystems (Zhuhai) Co., Ltd., Zhuhai, Guangdong 519031 (CN)
(72) Inventor: XIAO, Jiahua, Zhuhai, Guangdong 519031 (CN); LIU, Yue, Zhuhai, Guangdong 519031 (CN)
(74) Representative: Drexl, Janna
(86) International application number: PCT/CN2019/118314
(87) International publication number: WO 2020/244154

(56) References cited:
- EP-A1- 0 379 794
- EP-A1- 2 110 151
- WO-A1-01/12061
- CN-A- 101 822 866
- CN-A- 101 822 866
- CN-A- 106 102 816
- CN-A- 106 880 400
- CN-A- 106 880 400
- CN-A- 110 115 798
- CN-U- 208 541 677
- US-A1- 2002 045 925
- US-A1- 2016 339 204
- US-B1- 7 144 407
- US-B2- 9 174 030

## Description

### TECHNICAL FIELD

The present invention relates to the field of medical device technologies, and specifically to a balloon catheter.

### BACKGROUND

A balloon catheter is a terminal device for interventional procedures, and is mainly used in angioplasty, cardiovascular treatment, stent delivery, lumen dilation, tracheal and esophageal dilatation and treatment, high pressure dilation, treatment of neurovascular occlusion, and the like. A balloon catheter is inserted into blood vessels, lumen, trachea, esophagus, urethra, and the like, and a balloon is used for support. The flow distribution and flow rate distribution of a medium in the balloon during the treatment have a relatively large impact on the treatment effect.

International Patent Application WO 01/12061 A1 discloses a central venous catheter with heat exchange properties.

For a balloon catheter in the prior art, in a pressurized state, a medium flows through a catheter to enter a balloon cavity from an inlet and flows back through an outlet to complete circulation. The position of the inlet for the medium inside the balloon affects the flow rate distribution of the medium on the inner surface of the balloon. Due to the characteristics of the flow of the medium, when the medium is in contact with the inner surface of the balloon for heat exchange, there is a relatively small amount of effective heat exchange medium, resulting in incomplete heat exchange and therefore an inadequate therapeutic effect.

### SUMMARY

An objective of the present invention is to provide a balloon catheter, to solve the technical problem of incomplete heat exchange of a heat exchange medium in the prior art.

This objective is achieved by means of a balloon catheter according to appended claim 1. Preferred embodiments are detailed in the appended dependent claims.

To achieve the foregoing objective, the technical solution adopted in the present invention is as follows: A balloon catheter is provided, including a catheter and a balloon body sleeved on the catheter, where the balloon body includes an inner layer balloon and an outer layer balloon, the inner layer balloon and the outer layer balloon are both directly wrapped and fixed on the catheter, the inner layer balloon is located inside the outer layer balloon, a medium channel is formed between the inner layer balloon and the outer layer balloon, and an outer layer medium inlet hole and a medium backflow hole both in communication with the medium channel are provided in the catheter.

Further, according to the invention, an inner layer medium inlet hole in communication with the inner layer balloon is further provided in the catheter.

Further, according to the invention, a medium inlet tube and a medium backflow tube are provided in the catheter, the inner layer medium inlet hole and the outer layer medium inlet hole are both in communication with the medium inlet tube, and the medium backflow tube is in communication with the medium backflow hole.

Further, according to the invention, a control valve is disposed on the medium inlet tube, and the control valve is located between the inner layer medium inlet hole and the outer layer medium inlet hole.

Further, according to a preferred embodiment, an end of the medium inlet tube is a catheter terminal end, the outer layer medium inlet hole is located at one end of the outer layer balloon away from the catheter terminal end, and the inner layer medium inlet hole is located on a side of the outer layer medium inlet hole close to the catheter terminal end.

Further, according to a preferred embodiment, the medium backflow hole is located at the other end of the outer layer balloon close to the catheter terminal end.

Further, according to a preferred embodiment, a guidewire cavity is further disposed in the catheter.

Further, according to a preferred embodiment, the guidewire cavity is slidably connected to the catheter, and an end of the guidewire cavity away from a catheter terminal end is fixedly connected to an end of the outer layer balloon away from the catheter terminal end.

Further, according to a preferred embodiment, a marking ring is disposed inside the inner layer balloon or the outer layer balloon.

The beneficial effects of the balloon catheter provided in the present invention are as follows: Compared with the prior art, in the balloon catheter in the present invention, the double-layer balloon body is provided, so that a medium can flow in the medium channel between the inner layer balloon and the outer layer balloon, the medium flows into the medium channel through the outer layer medium inlet hole, and then the medium in the medium channel flows out through the medium backflow hole to implement circulation. In this way, more medium can exchange heat on the inner surface of the outer layer balloon and the flow rate distribution is relatively uniform, to increase the flowing quality of the medium on the surface of the balloon body, thereby improving the heat exchange efficiency.

### BRIEF DESCRIPTION OF THE DRAWINGS

To describe the technical solutions in specific embodiments of the present invention or the prior art more clearly, the following briefly introduces the accompanying drawings required for describing the specific embodiments or the prior art. Apparently, the accompanying drawings in the following description show some embodiments of the present invention, and a person of ordinary skill in the art may still derive other drawings from these accompanying drawings without creative efforts.
FIG. 1 is a schematic structural diagram of a balloon catheter according to an embodiment of the present invention;
FIG. 2 is a schematic diagram of the flow of a medium in a balloon catheter according to an embodiment of the present invention;
FIG. 3 is a schematic structural sectional view 1 of a balloon catheter according to an embodiment of the present invention; and
FIG. 4 is a schematic structural sectional view 2 of a balloon catheter according to an embodiment of the present invention.

Reference numerals:
1. catheter; 2. balloon body; 11. outer layer medium inlet hole; 12. medium backflow hole; 13. inner layer medium inlet hole; 14. medium inlet tube; 15. medium backflow tube; 16. guidewire cavity; 17. catheter terminal end; 18. fixing end portion; 19. slidable tube body; 141. control valve; 21. inner layer balloon; 22. outer layer balloon; 23. medium channel; and 24. marking ring.

### DETAILED DESCRIPTION

The following clearly and completely describes the technical solutions of the present invention with reference to the accompanying drawings. Apparently, the described embodiments are merely some rather than all of the embodiments of the present invention.

In the description of the present invention, it needs to be understood that orientation or location relationships indicated by terms "center", "up", "down", "left", "right", "vertical", "horizontal", "inside", and "outside" are based on orientation or location relationships shown in the accompanying drawings, and are only used to facilitate description of the present invention and simplify description, but are not used to indicate or imply that the apparatuses or elements must have specific orientations or are constructed and operated by using specific orientations, and therefore, cannot be understood as a limitation to the present invention. In addition, the terms "first", "second", and "third" are used only for description, but are not intended to indicate or imply relative importance.

In the description of the present invention, it needs to be noted that unless otherwise expressly specified and defined, "mounted", "connected", and "connection", should be understood in a broad sense, for example, fixedly connected, detachably connected or integrally connected; or mechanically connected or electrically connected; or connected directly or indirectly through an intermediate, or two elements communicated internally. For a person of ordinary skill in the art, specific meanings of the terms in the present invention should be understood according to specific conditions.

In addition, the technical features involved in different embodiments of the present invention described below can be combined with each other as long as they do not constitute a conflict between them.

### Embodiment 1

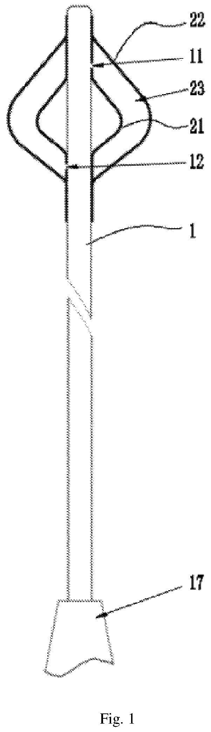

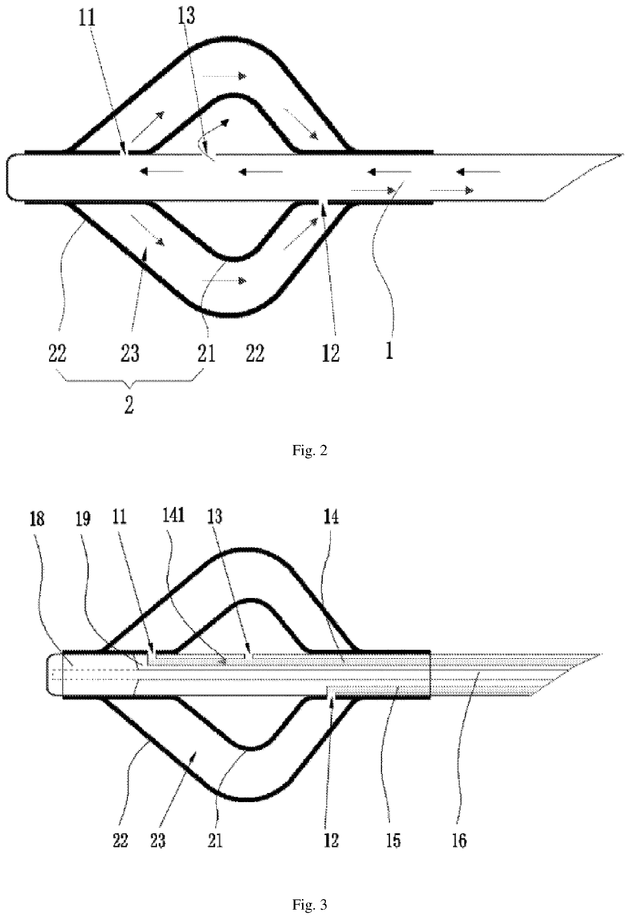

Referring to FIG. 1 and FIG. 2 together, a balloon catheter provided in the present invention is described. The balloon catheter includes a catheter 1 and a balloon body 2 sleeved on the catheter 1. The balloon body 2 includes an inner layer balloon 21 and an outer layer balloon 22. The inner layer balloon 21 and the outer layer balloon 22 are both directly wrapped and fixed on the catheter 1. The inner layer balloon 21 is located inside the outer layer balloon 22. A medium channel 23 is formed between the inner layer balloon 21 and the outer layer balloon 22. An outer layer medium inlet hole 11 and a medium backflow hole 12 both in communication with the medium channel 23 are provided in the catheter 1.

In the balloon catheter provided in the present invention, compared with the prior art, the double-layer balloon body 2 is provided, so that a medium can flow in the medium channel 23 between the inner layer balloon 21 and the outer layer balloon 22, the medium flows into the medium channel 23 through the outer layer medium inlet hole 11, and then the medium in the medium channel 23 flows out through the medium backflow hole 12 to implement circulation of the medium. In this way, more medium can exchange heat on the inner surface of the outer layer balloon 22 and the flow rate distribution is relatively uniform, to increase the flowing quality of the medium on the surface of the balloon body 2, thereby improving the heat exchange efficiency.

Specifically, the balloon body 2 is directly sleeved on the catheter 1. The balloon body 2 is generally disposed at a terminal end of the catheter 1. The balloon body 2 is expandable under the filling of the medium, to fill and support narrow places such as blood vessels, trachea and urethra. Middle parts of the inner layer balloon 21 and the outer layer balloon 22 can both swell after the medium is filled, the inner layer balloon 21 and the outer layer balloon 22 are generally the same in shape, and there is the same distance between the outer surface of the inner layer balloon 21 and the inner surface of the outer layer balloon 22, to ensure that the entire medium channel 23 has the same channel size, thereby ensuring relatively uniform flow rate distribution of the medium. Certainly, according to an actual case and specific requirements, in other embodiments of the present invention, the inner layer balloon 21 and the outer layer balloon 22 may have different shapes. This is not uniquely limited herein.

Further, referring to FIG. 1 to FIG. 3 together, according to the present invention, an inner layer medium inlet hole 13 in communication with the inner layer balloon 21 is further provided in the catheter 1. Specifically, the medium may flow through the inner layer medium inlet hole 13 into the inner layer balloon 21, to ensure that the medium can fill the inner layer balloon 21. The medium filling the inner layer balloon 21 and the medium filling the outer layer balloon 22 are generally the same medium. A through hole is directly provided in a sidewall of the catheter 1, so that the medium located in the catheter 1 may flow into the inner layer balloon 21.

Preferably, there may be a plurality of inner layer medium inlet holes 13, a plurality of outer layer medium inlet holes 11, and a plurality of medium backflow holes 12, which are evenly distributed in the sidewall of the catheter 1, so that the speed at which the medium in the catheter 1 flows into the balloon body 2 can be increased, and it can be ensured that the flow rate of the medium in the outer layer balloon 22 is uniform.

Further, referring to FIG. 1 to FIG. 3, according to the present invention, a medium inlet tube 14 and a medium backflow tube 15 are provided in the catheter 1. The inner layer medium inlet hole 13 and the outer layer medium inlet hole 11 are both in communication with the medium inlet tube 14. The medium backflow tube 15 is in communication with the medium backflow hole 12. Specifically, the medium inlet tube 14 and the medium backflow tube 15 are two separate tube bodies, and the medium inlet tube 14 and the medium backflow tube 15 are both in direct communication with an outer side of the catheter 1. The medium inlet tube 14 may be in direct communication with the inner layer medium inlet hole 13 and the outer layer medium inlet hole 11. Both the medium in the inner layer balloon 21 and the medium in the outer layer balloon 22 are transported through the medium inlet tube 14. The medium in the outer layer balloon 22 flows through the medium channel 23 and flows out through the medium backflow hole 12 to the medium backflow tube 15 to implement the circulation of all the medium. Certainly, according to an actual case and specific requirements, in other embodiments of the present invention, the medium inlet tube 14 may be only in communication with the outer layer medium inlet hole 11. The medium required for the inner layer balloon 21 may be directly transported by a separate tube body. This is not uniquely limited herein.

Further, referring to FIG. 1 to FIG. 3, according to the present invention, a control valve 141 is disposed on the medium inlet tube 14. The control valve 141 is located between the inner layer medium inlet hole 13 and the outer layer medium inlet hole 11. Specifically, the control valve 141 can control the flow direction of the medium, to ensure that the medium first flows from the medium inlet tube 14 to the inner layer balloon 21, and then, when the pressure in the inner layer balloon 21 is greater than a threshold, the control valve 141 is opened to allow the medium to flow to the outer layer medium inlet hole 11 to fill the outer layer balloon 22, thereby avoiding the problem that the medium directly enters the outer layer balloon 22 for circulation and fails to fill the inner layer balloon 21.

Further, referring to FIG. 1 to FIG. 3, in a specific implementation of the balloon catheter provided in the present invention, the control valve 141 is a throttle check valve. Specifically, a throttle valve and a check valve may be combined into a throttle check valve. The throttle valve can open under the action of pressure, and the throttle valve allows one direction, so that the medium can only flow through the inner layer medium inlet hole 13 to enter the outer layer medium inlet hole 11. The backflow of the medium can be effectively avoided. The throttle check valve can ensure the use stability of the balloon catheter, to ensure that the medium fills the inner layer balloon 21 before the medium can fill the outer layer balloon 22, thereby avoiding direct backflow of the medium in the outer layer balloon 22.

Further, referring to FIG. 1 to FIG. 3, in a specific implementation of the balloon catheter provided in the present invention, the medium inlet tube 14 and the medium backflow tube 15 are located on two opposite sides on an inner side of the catheter 1. Specifically, the medium inlet tube 14 and the medium backflow tube 15 are located on the inner side of the catheter 1, and the medium inlet tube 14 and the medium backflow tube 15 are both located on a side of the catheter 1 close to a sidewall, so that connections between the inner layer medium inlet hole 13, the outer layer medium inlet hole 11, and the medium backflow hole 12 can be conveniently implemented, and the connections can be implemented by directly opening through holes in the medium inlet tube 14 or the medium backflow tube 15. Certainly, according to an actual case and specific requirements, in other embodiments of the present invention, the medium inlet tube 14 and the medium backflow tube 15 may be directly filled inside the catheter 1, or the medium inlet tube 14 and the medium backflow tube 15 may be tubes independent of the catheter 1, or all channels are designed into a composite multi-cavity tube. This is not uniquely limited herein.

Further, referring to FIG. 1 to FIG. 3, in a specific implementation of the balloon catheter provided in the present invention, an end of the medium inlet tube 14 is a catheter terminal end 17. The outer layer medium inlet hole 11 is located at one end of the outer layer balloon 22 away from the catheter terminal end 17. The inner layer medium inlet hole 13 is located on a side of the outer layer medium inlet hole 11 close to the catheter terminal end 17. Specifically, an end of the catheter 1 is the catheter terminal end 17. The catheter terminal end 17 may be an inlet end for the medium and an insertion end for a guidewire. The inner layer medium inlet hole 13 is located near the catheter terminal end 17. That is, the medium fills the inner layer balloon 21 before the medium can fill the outer layer balloon 22, to avoid that all the medium flows into the outer layer balloon 22 to flow out through the medium backflow hole 12, thereby ensuring that both the inner layer and the outer layer of the balloon body 2 can be filled. Certainly, according to an actual case and specific requirements, in other embodiments of the present invention, when an inner layer medium tube is separately disposed, the positions of the inner layer medium inlet hole 13 and the outer layer medium inlet hole 11 may be adjusted as required. This is not uniquely limited herein.

Further, referring to FIG. 1 to FIG. 3, in a specific implementation of the balloon catheter provided in the present invention, the medium backflow hole 12 is located at the other end of the outer layer balloon 22 close to the catheter terminal end 17. Specifically, the medium backflow hole 12 and the outer layer medium inlet hole 11 are separately are located at two opposite ends of the outer layer balloon 22, to ensure that the medium can form complete circulation in the outer layer balloon 22, thereby avoiding that the medium fails to completely fill the outer layer balloon 22, so that it is avoided that the support and fixation are inadequate because the outer layer balloon 22 fails to fully expand. Certainly, according to an actual case and specific requirements, in other embodiments of the present invention, a throttle valve may be disposed at the medium backflow hole 12. The medium can flow back through the medium backflow hole 12 only when the pressure in the outer layer balloon 22 is greater than a particular degree. This is not uniquely limited herein.

Further, referring to FIG. 1 to FIG. 3, in a specific implementation of the balloon catheter provided in the present invention, a guidewire cavity 16 is further disposed in the catheter 1. Specifically, the guidewire cavity 16 has assistant functions such as threading a guidewire and introducing a liquid. The guidewire cavity 16 is usually located at the middle part of the catheter 1, the medium inlet tube 14 and the medium backflow tube 15 are both located on two sides of the guidewire cavity 16, and a space for the guidewire cavity 16 is reserved in the catheter 1, to allow for increased applicability and increased functionality of the balloon catheter.

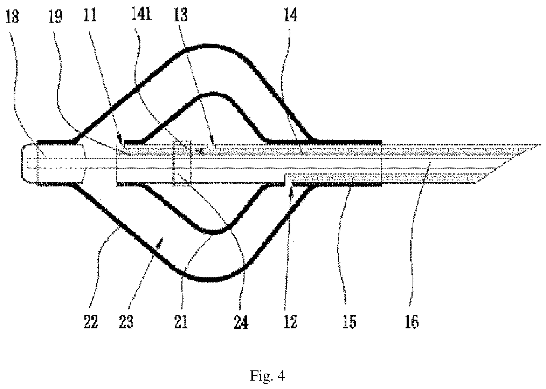

Further, referring to FIG. 3 and FIG. 4, in a specific implementation of the balloon catheter provided in the present invention, the guidewire cavity 16 is slidably connected to the catheter 1, and an end of the guidewire cavity 16 away from the catheter terminal end 17 is fixedly connected to one end of the outer layer balloon 22 away from the catheter terminal end 17. Specifically, the catheter 1 has a long tube body. The guidewire cavity 16 is provided inside the catheter 1. The guidewire cavity 16 may slide relatively inside the catheter 1. The end of the guidewire cavity 16 away from the catheter terminal end 17 may be directly fixedly connected to the end of the outer layer balloon 22 away from the catheter terminal end 17. The other end of the outer layer balloon 22 close to the catheter terminal end 17 is fixedly connected to the catheter 1, so that a spacing between two ends of the outer layer balloon 22 can be changed. When the spacing between the two ends of the outer layer balloon 22 increases, the outer layer balloon 22 has an increased side length and is attached to the outer side of the catheter 1, to further facilitate interventional treatment with the entire balloon catheter. When the spacing between the two ends of the outer layer balloon 22 decreases, the outer layer balloon 22 can be filled to provide support.

Further, referring to FIG. 3 and FIG. 4, in a specific implementation of the balloon catheter provided in the present invention, a marking ring 24 is disposed inside the inner layer balloon 21 or the outer layer balloon 22. Specifically, the marking ring 24 is disposed inside the balloon body 2 and may mark the position of the balloon body 2 to facilitate observation from outside. The marking ring 24 is generally disposed inside the inner layer balloon 21 or the outer layer balloon 22. The marking ring 24 is annularly disposed on the outer side of the catheter 1 to implement the fixation of the marking ring 24. The material of the marking ring 24 is usually a platinum-iridium alloy or another alloy material that can implement marking.

### Embodiment 2

In another embodiment not forming part of the present invention as claimed, referring to FIG. 1 to FIG. 3, differences between this embodiment and Embodiment 1 are as follows: A medium inlet tube 14, a medium backflow tube 15, and an inner layer medium tube (not shown in the figure) are disposed in the catheter 1, the inner layer medium inlet hole 13 is in communication with the inner layer medium tube, the outer layer medium inlet hole 11 is in communication with the medium inlet tube 14, and the medium backflow tube 15 is in communication with the medium backflow hole 12. Specifically, the medium inlet tube 14, the medium backflow tube 15, and the inner layer medium tube are all separate tube bodies, and the inner layer medium inlet hole 13 is in communication with the inner layer medium tube. Because the inner layer medium tube and the medium inlet tube 14 are two separate tube bodies, it is not necessary to arrange the control valve 141 to control the flow rate, so that the control method is simpler, and a medium can be directly transported for the inner layer balloon 21 and the outer layer balloon 22. The medium in the outer layer balloon 22 flows through the medium channel 23 and flows out through the medium backflow hole 12 to the medium backflow tube 15 to implement the circulation of all the medium.

Obviously, the foregoing embodiments are merely examples for clear description, rather than a limitation to implementations. For a person of ordinary skill in the art, other changes or variations in different forms may also be made based on the foregoing description. All implementations cannot and do not need to be exhaustively listed herein. Obvious changes or variations that are derived there from still fall within the protection scope of the present invention, which is defined solely by the appended claims.

## Claims

1. A balloon catheter, comprising a catheter (1) and a balloon body (2) sleeved on the catheter (1), wherein the balloon body (2) comprises an inner layer balloon (21) and an outer layer balloon (22), the inner layer balloon (21) and the outer layer balloon (22) are both directly wrapped and fixed on the catheter (1), the inner layer balloon (21) is located inside the outer layer balloon (22), a medium channel (23) is formed between the inner layer balloon (21) and the outer layer balloon (22), and an outer layer medium inlet hole (11) and a medium backflow hole (12) both in communication with the medium channel (23) are provided in the catheter (1)
wherein an inner layer medium inlet hole (13) in communication with the inner layer balloon (21) is further provided in the catheter (1),
a medium inlet tube (14) and a medium backflow tube (15) are provided in the catheter (1), the inner layer medium inlet hole (13) and the outer layer medium inlet hole (11) are both in communication with the medium inlet tube (14), and the medium backflow tube (15) is in communication with the medium backflow hole (12),
a control valve (141) is disposed on the medium inlet tube (14), and the control valve (141) is located between the inner layer medium inlet hole (13) and the outer layer medium inlet hole (11).

2. The balloon catheter according to claim 1, wherein an end of the medium inlet tube (14) is a catheter terminal end (17), the outer layer medium inlet hole (11) is located at one end of the outer layer balloon (22) away from the catheter terminal end (17), and the inner layer medium inlet hole (13) is located on a side of the outer layer medium inlet hole (11) close to the catheter terminal end (17).

3. The balloon catheter according to claim 2, wherein the medium backflow hole (12) is located at the other end of the outer layer balloon (22) close to the catheter terminal end (17).

4. The balloon catheter according to claim 1, wherein a guidewire tube (16) is further disposed in the catheter (1).

5. The balloon catheter according to claim 4, wherein the guidewire tube (16) is slidably connected to the catheter (1), and an end of the guidewire tube (16) away from a catheter terminal end (17) is fixedly connected to an end of the outer layer balloon (22) away from the catheter terminal end (17).

6. The balloon catheter according to claim 1, wherein a marking ring (24) is disposed inside the inner layer balloon (21) or the outer layer balloon (22).

## Patentansprüche

1. Ein Ballonkatheter umfassend einen Katheter (1) und einen Ballonkörper (2) der über den Katheter (1) gestülpt ist, wobei der Ballonkörper (2) eine innere Ballonschicht (21) und eine äußere Ballonschicht (22) umfasst, die innere Ballonschicht (21) und die äußere Ballonschicht (22) sind beide direkt auf den Katheter gewickelt und befestigt, die innere Ballonschicht (21) ist innerhalb der äußeren Ballonschicht (22) lokalisiert, ein Mediumkanal (23) wird zwischen der inneren Ballonschicht (21) und der äußeren Ballonschicht (22) gebildet und eine Außenschicht-Medium-Einlassöffnung (11) und eine Medium-Rückflussöffnung (12), die beide in Verbindung mit dem Mediumkanal (23) stehen, werden in dem Katheter (1) bereitgestellt, wobei
eine Innenschicht-Medium-Einlassöffnung (13), die in Verbindung mit der inneren Ballonschicht (21) steht, ferner in dem Katheter (1) bereitgestellt wird,
ein Medium-Einlassrohr (14) und ein Medium-Rückflussrohr (15) werden in dem Katheter (1) bereitgestellt, die Innenschicht-Medium-Einlassöffnung (13) und die Außenschicht-Medium-Einlassöffnung (11) stehen beide in Verbindung mit dem Medium Einlassrohr (14), und das Medium-Rückflussrohr (15) steht mit der Medium-Rückflussöffnung (12) in Verbindung,
ein Kontrollventil (141) ist auf dem Medium-Einlassrohr (14) angebracht, und das Kontrollventil (141) ist zwischen der Innenschicht-Medium-Einlassöffnung (13) und der Außenschicht Medium-Einlassöffnung (11) lokalisiert.

2. Der Ballonkatheter nach Anspruch 1, wobei ein Ende des Medium-Einlassrohrs (14) ein terminales Ende des Katheters (17) ist, die Außenschicht-Medium-Einlassöffnung (11) ist an einem Ende der äußeren Ballonschicht (22) lokalisiert weg von dem terminalen Ende des Katheters (17) und die Innenschicht-Medium-Einlassöffnung (13) ist auf einer Seite der Außenschicht-Medium-Einlassöffnung (11) nahe des terminalen Endes des Katheters (17) lokalisiert.

3. Der Ballonkatheter nach Anspruch 2, wobei die Medium-Rückflussöffnung (12) am anderen Ende der äußeren Ballonschicht (22) in der Nähe des terminalen Endes des Katheters (17) lokalisiert ist.

4. Der Ballonkatheter nach Anspruch 1, wobei ferner ein Führungsdrahtrohr (16) in dem Katheter (1) angebracht ist.

5. Der Ballonkatheter nach Anspruch 4, wobei das Führungsdrahtrohr (16) verschiebbar mit dem Katheter (1) verbunden ist und ein Ende des Führungsdrahtrohrs (16) weg von einem terminalen Ende des Katheters (17) fest mit einem Ende der äußeren Ballonschicht (22) weg vom terminalen Ende des Katheters (17) verbunden ist.

6. Der Ballonkatheter nach Anspruch 1, wobei ein Markierungsring (24) innerhalb der inneren Ballonschicht (21) oder der äußeren Ballonschicht (22) angebracht ist.

## Revendications

1. Cathéter à ballonnet, comprenant un cathéter (1) et un corps de ballonnet (2) enfilé sur le cathéter (1), dans lequel le corps de ballonnet (2) comprend un ballonnet de couche interne (21) et un ballonnet de couche externe (22), le ballonnet de couche interne (21) et le ballonnet de couche externe (22) sont tous les deux directement enroulés et fixés sur le cathéter (1), le ballonnet de couche interne (21) est situé à l'intérieur du ballonnet de couche externe (22), un canal de milieu (23) est formé entre le ballonnet de couche interne (21) et le ballonnet de couche externe (22), et un trou d'entrée de milieu de couche externe (11) et un trou de reflux de milieu (12) tous les deux en communication avec le canal de milieu (23) sont prévus dans le cathéter (1)
dans lequel un trou d'entrée de milieu de couche interne (13) en communication avec le ballonnet de couche interne (21) est en outre prévu dans le cathéter (1),
un tube d'entrée de milieu (14) et un tube de reflux de milieu (15) sont prévus dans le cathéter (1), le trou d'entrée de milieu de couche interne (13) et le trou d'entrée de milieu de couche externe (11) sont tous les deux en communication avec le tube d'entrée de milieu (14), et le tube de reflux de milieu (15) est en communication avec le trou de reflux de milieu (12),
une soupape de contrôle (141) est disposée sur le tube d'entrée de milieu (14), et la soupape de contrôle (141) est située entre le trou d'entrée de milieu de couche interne (13) et le trou d'entrée de milieu de couche externe (11).

2. Cathéter à ballonnet selon la revendication 1, dans lequel une extrémité du tube d'entrée de milieu (14) est une extrémité terminale de cathéter (17), le trou d'entrée de milieu de couche externe (11) est situé au niveau d'une extrémité du ballonnet de couche externe (22) à l'écart de l'extrémité terminale de cathéter (17), et le trou d'entrée de milieu de couche interne (13) est situé sur un côté du trou d'entrée de milieu de couche externe (11) à proximité de l'extrémité terminale de cathéter (17).

3. Cathéter à ballonnet selon la revendication 2, dans lequel le trou de reflux de milieu (12) est situé au niveau de l'autre extrémité du ballonnet de couche externe (22) à proximité de l'extrémité terminale de cathéter (17).

4. Cathéter à ballonnet selon la revendication 1, dans lequel un tube de fil-guide (16) est en outre disposé dans le cathéter (1).

5. Cathéter à ballonnet selon la revendication 4, dans lequel le tube de fil-guide (16) est relié de manière coulissante au cathéter (1), et une extrémité du tube de fil-guide (16) à l'écart d'une extrémité terminale de cathéter (17) est reliée de manière fixe à une extrémité du ballonnet de couche externe (22) à l'écart de l'extrémité terminale de cathéter (17).

6. Cathéter à ballonnet selon la revendication 1, dans lequel une bague de repérage (24) est disposée à l'intérieur du ballonnet de couche interne (21) ou du ballonnet de couche externe (22).
